# EUROPEAN PATENT SPECIFICATION

(11) **EP 3 600 547 B1**
(45) Date of publication and mention of the grant of the patent: **23.12.2020**
(21) Application number: 18717250.7
(22) Date of filing: 26.03.2018
(51) Int. Cl.: A61N 5/10, A61B 17/34, A61B 90/98

(54) **SYSTEM FOR POSITIONING MEDICAL NEEDLES**
SYSTEM ZUR POSITIONIERUNG MEDIZINISCHER NADELN
SYSTÈME DE POSITIONNEMENT D'AIGUILLES MÉDICALES

(30) Priority: 30.03.2017 EP 17382162
(43) Date of publication of application: 05.02.2020
(73) Proprietor: Servicio Cántabro de Salud, 39008 Santander (ES); Universidad de Cantabria, 39005 Santander (ES)
(72) Inventor: PRADA GÓMEZ, Pedro José, 39008 Santander (ES); RABA DÍEZ, Juan Ignacio, 39008 Santander (ES); RODRÍGUEZ COBO, Luis, 39005 Santander (ES); VALDIANDE GUTIERREZ, José Julián, 39005 Santander (ES); COBO GARCÍA, Adolfo, 39005 Santander (ES); CONDE PORTILLA, Olga María, 39005 Santander (ES); LÓPEZ HIGUERA, José Miguel, 39005 Santander (ES)
(74) Representative: Balder IP Law, S.L.
(86) International application number: PCT/EP2018/057681
(87) International publication number: WO 2018/178031

(56) References cited:
- US-A1- 2007 043 291
- US-A1- 2010 019 918
- US-A1- 2017 014 192
- US-A1- 2017 014 641

## Description

### TECHNICAL FIELD

The present invention relates to the field of systems for positioning medical implants. In particular, it relates to systems for radiation oncology technics, such as brachytherapy.

### STATE OF THE ART

Cancer is often treated with a combination of therapies. One of these therapies is radiation therapy (radiotherapy). Brachytherapy is a form of radiotherapy where a sealed radiation source is placed in the body -animal body in general- at or next to the area requiring treatment. The radioactive source is placed using catheters or hollow needles, which are inserted within the body towards the target location. The radioactive source may be a high dose rate (HDR) energy emitting source or a low dose rate (LDR) one. LDR radioactive sources are permanently implanted into the body tissue. Brachytherapy is commonly used as an effective treatment for cervical, prostate, breast, and skin cancer and can also be used to treat tumors in many other body sites.

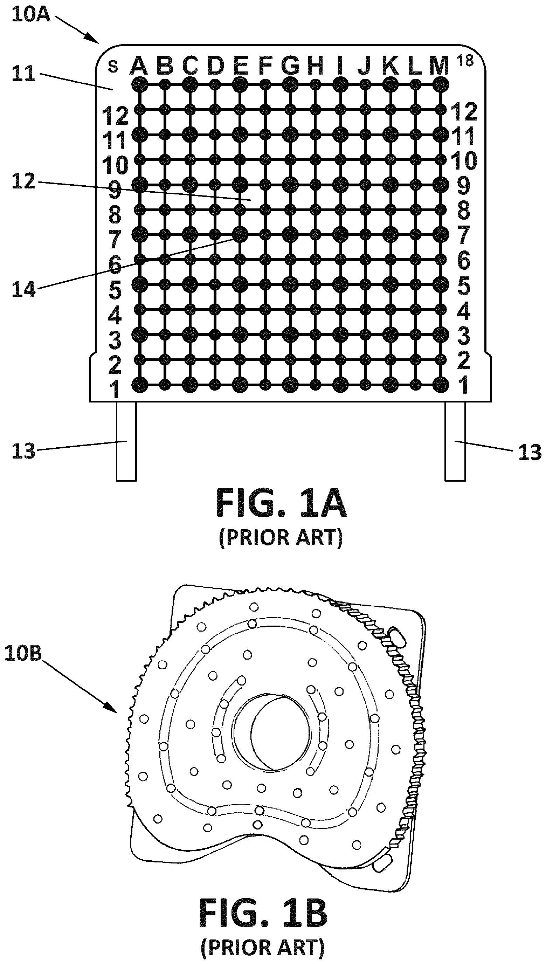

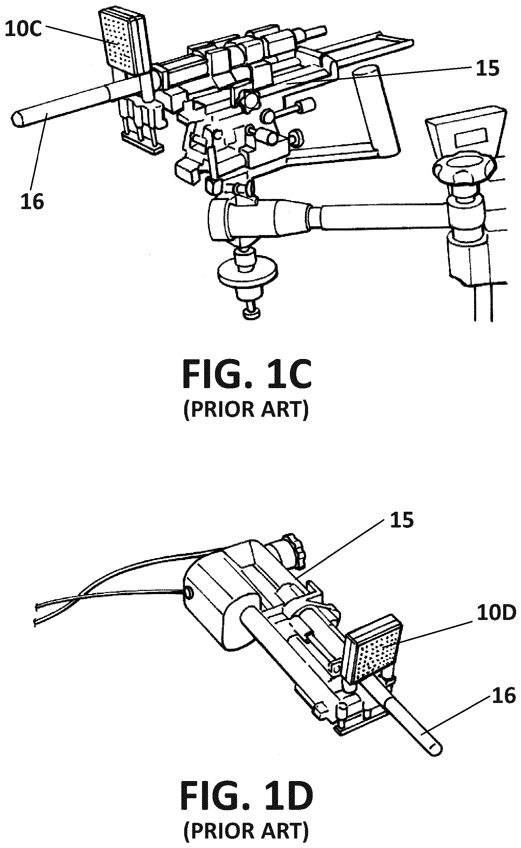

In order to insert needles within the body across the skin, template devices are conventionally used. Brachytherapy template devices typically include fixation means, such as a frame or one or more attachments, and a needle guiding component for guiding the needles into the body towards the target position. Figure 1A shows a conventional passive brachytherapy grid or template device 10A having two attachments 13 for fixing the device to additional medical equipment on which template devices are typically mounted. As a matter of example, figures 1C and 1D show respective steppers 15 on which template devices 10C 10D are usually mounted. The shown steppers 15 also have an ultrasound probe 16 coupled thereto.

Turing back to figure 1A, the template device may include a frame providing physical integrity and a guide plate incluiding a plurality of apertures, openings or through holes for guiding the needles towards the desired location in the body. The template device shown in figure 1A has the guiding component 12 integrated within the frame 11. The apertures 14 typically form an x-y array (cartesian array), that is to say, apertures 14 extend in rows oriented in the x direction and in columns oriented in the y direction. The device 10A in figure 1A has 13 rows and 13 columns, there rows being identified by numbers (1, 2, ...12) and columns being identified by letters (A, B, ...M), but any other amount of rows and columns could be chosen. Each aperture is sized to receive one needle. Thus, each aperture corresponds to a location in the x and y directions on a cross section of a body part parallely disposed to the guiding component.

Figure 1B shows another conventional passive brachytherapy grid or template device 10B, in which the guiding component is also integrated within the frame. However, the apertures in this device 10B does not form a cartesian array (that is to say, they do not form an x-y array). Besides, in this case the apertures are not referenced. Even if they were, for example by assigning a number to each aperture, identification of needles becomes difficult due to the absence of regular pattern.

The shown conventional templates do not ensure that the radiation sources are placed in the desired location as prescribed by the treatment plan with the accuracy required for a correct treatment. Even very small deviations in the placement of radiation sources may cause that the remaining tumor margin is not receiving the appropriate radiation and that healthy tissue is receiving unwanted radiation.

Developments have been made in brachytherapy templates in order to improve the reliability of the needles placement -and therefore radiation source- in the exact target location. US7527593B2 discloses a guide plate for inserting needles into a body through a plurality of openings disposed in the guide plate. For each of the openings, there is a sensor arrangement for detecting the presence of a needle inserted through the opening. Each sensor is implemented by means of a LED and a photodetector, in such a way that when a needle is inserted through the opening, the needle blocks the light flow from the LED, thus detecting the presence of the needle due to the absence of light. Or each sensor is implemented by means of an inductor whose inductance is changed as the needle passes through the aperture. The sensors of the guide plate are connected to a display showing which apertures in the guide plate have needles therein and which apertures don't. Thus, the locations where needles must be inserted are identified with a first color on the display and all other locations with a second color. Once it has been detected that a needle is inserted through the guide plate, the color of the corresponding location on the display changes from the first color to the second color, thus indicating that a needle is already placed at the correct location. Similarly, US2010/0019918A1 discloses a probe insertion template that uses light sources to draw user attention to an aperture through which a probe must be inserted. US2017/014192A1 discloses systems for tracking an insertion of an interventional tool into an anatomical region. US2017/0014641 A1 discloses a template device for brachytherapy and other procedures having apertures allowing passage of a needle. Some apertures have a sensing device that emits a signal in response to sensing a needle in the corresponding aperture.

Another shortcoming of conventional brachytherapy template devices is the determination of how deep the needle has been inserted into the body tissue. The depth of the needle is a feature as important as the needle location in order to ensure a correct treatment. Nowadays the needle penetration is measured by different relatively inaccurate techniques. The simplest one is by manually measuring the length of a needle outside the body tissue, thus calculating, by a mere subtraction, the amount of length disposed within the body tissue, and thus the penetration of the needle. The manual measuring is slow and tedious, and subject to potential human mistakes. A different technique is suggested in US8764619B2. US8764619B2 discloses a brachytherapy needle fixation system including needles having visualization features (external geometric patterns) for determining the position and orientation of the needle relative to an electromagnetic imaging device. Visualization features are visualized by means of an imaging device that generates electromagnetic energy. Energy generated by the imaging device strikes the needle and is reflected by the features of the needle as radiated energy that is received by the imaging device. However, in order to try to clearly identify the geometry of the needle, the imaging device has to be installed in a transversal plane with respect to the template device. For example, in a treatment for breast cancer, in which the needles are inserted into the body tissue through a template device disposed on a side of the body, the imaging device needs to be disposed on the breast (frontal part of the body). This complicates the application of this technique to such an extent that it often becomes inadvisable, since correct detection of the needles from an accessible plane of the patient becomes impossible. Besides, inserting a needle having external geometric patterns into human tissue is more complicated than inserting a smooth needle (i.e. a needle having even surface). The manufacturing of needles having external geometric patterns involves also a more complex industrial process.

### DESCRIPTION OF THE INVENTION

The system and device described in the present disclosure intend to solve the shortcomings of prior-art systems and devices. The invention is as defined in the appended claims.

The invention provides a simple mechanism to facilitate the insertion of needles into body tissue with accurate control of both position and penetration depth. The invention is based on a guide plate having a plurality of apertures for inserting needles and a detection block permitting both the detection of the presence of needles inserted through the apertures and the penetration depth of each of the inserted needles. The detection block may be implemented by sensing means. The detection block may simultaneously detect the presence of several needles. The penetration depth of a needle may be measured using a probe to be inserted along a needle in turn inserted into an aperture of the guide plate. Besides, the guide plate may provide the person inserting the needles (doctor, nurse, physician or, or in general, medical staff) with a visual guidance at the insertion area in order to prevent human mistakes. This may be achieved by means of an illumination block implemented with lighting means disposed at each of the apertures of the guide plate. The illumination block may be implemented as an electro-optic system. The status of each lighting means indicates whether a needle must be inserted through the corresponding aperture or not. The invention also provides a system including the guide plate, for guiding in the insertion of needles into body tissue.

The guide plate may be used in combination with conventional passive templates, that is to say, with conventional templates not enabling an accurate control of position and/or penetration of needles into body tissue, or may be integrally comprised or embedded in templates during its manufacturing process, the templates thus becoming autonomous active templates, that is to say, not requiring the use of additional guide plates.

The invention relates to a system for inserting needles into a I body as defined in the appended claims, comprising:
a guide plate comprising a plurality of apertures, each aperture being configured to receive a needle to be inserted into a body, wherein at least some apertures of said plurality of apertures have a respective detector associated thereto, each detector comprising a resonant circuit for detecting the presence of a needle inserted into a respective aperture, said resonant circuit being configured to modify its resonant frequency when the needle is inserted into the aperture;
a probe for measuring, together with the detector comprised in an aperture, the penetration depth of a needle inserted into said aperture, each detector being configured to track changes in its resonant frequency produced when said probe.is inserted along the needle in turn inserted into the aperture; and control electronics for interrogating each detector and for determining the presence or absence of a needle in a corresponding aperture and for determining the penetration depth of a detected needle from said tracked changes in the resonant frequency of a corresponding detector.

In embodiments of the invention, the probe comprises along its longitudinal axis a plurality of portions of a first material and a plurality of portions of a second material, wherein portions of said first material alternate with portions of said second material, said first material being capable of modifying the resonant frequency of the detector associated to the aperture into which the needle is inserted, said second material not modifying the resonant frequency of said detector, the penetration depth of the needle being determined by counting the number of times the resonant frequency of said detector is modified as the probe slides along the needle until the probe reaches the distal end of the needle.

In embodiments of the invention, the needle to be received by an aperture comprises said first material.

In embodiments of the invention, the needle to be received by an aperture is configured to receive an additional element or device comprising said first material.

In embodiments of the invention, the probe comprises alternate portions of said first material and said second material, said portions being of equal length.

In embodiments of the invention, each resonant circuit comprises a coil and a capacitive element, wherein the nominal value of either said coil or said capacitive element is altered when a needle traverses said aperture.

In embodiments of the invention, the nominal value of said coil is altered when a needle traverses said aperture, the nominal value of said capacitive element remaining fixed.

In embodiments of the invention, the first material is a magnetic material and said second material is a material with no magnetic response.

In embodiments of the invention, the guide plate further comprises a light indicator associated to each aperture in the guide plate, each light indicator being configured to indicate a user of the system, prior the insertion of a needle, in which aperture said needle should be inserted and/or, after insertion of a needle, whether the needle has been correctly inserted according to a schedule previously planned, and/or configured to emit a light signal prior to inserting a needle and a modified light signal once a needle has been inserted, said modified light signal being indicative of the correctness or incorrectness location of the inserted needle.

In embodiments of the invention, the light indicator associated to each aperture in the guide plate is at least one light emitting diode (LED).

In embodiments of the invention, the guide plate further comprises interface means for connection with other equipment.

In embodiments of the invention, the guide plate is comprised in a brachytherapy template device.

An aspect of the disclosure relates to a guide plate, which can be used in a system according to the invention, for guiding the insertion of needles into a body, the guide plate comprising: a plurality of apertures, each aperture being configured for receiving a needle; wherein at least some apertures of said plurality of apertures have a respective detector associated thereto, each detector comprising a resonant circuit for detecting the presence of a needle inserted into a respective aperture, said resonant circuit being configured to modify its resonant frequency when the needle is inserted into the aperture; wherein each detector associated to a corresponding aperture is further configured to measure the penetration depth of a needle inserted into said aperture by tracking changes in the resonant frequency of said detector produced when a probe is inserted along the needle in turn inserted into said aperture.

In embodiments of the invention, the guide plate further comprises a light indicator associated to each aperture in the guide plate, each light indicator being configured to indicate a user of the system, prior the insertion of a needle, in which aperture said needle should be inserted and, after insertion of a needle, whether the needle has been correctly inserted according to a schedule previously planned.

Disclosed herein is also a brachytherapy template device, which can be used in a system according to the invention, comprising the guide plate of embodiments of the invention.

The system of the aspects of the invention are particularly applicable for use in brachytherapy as apparent from the present description. However, it is to be appreciated that the invention will also find application in conjunction with other therapeutic treatments, such as positioning other treatment sources, performing biopsies and the like. Besides, the device can be used to implant or apply different radioactive sources into various different tissues, such as breast tissue, prostate tissue and others.

The system of the invention provides accurate control of the position and penetration of needles into a body tissue. It also provides quick feedback to the person inserting the needles, so that quick reaction in the event of an erroneous insertion can be made. Thanks to this accurate control of position and penetration, any shape of guide plate having any arrangement of apertures may be used, rather than the conventional x-y arrays traditionally used because they are considered easier to follow a scheduled plan. So, irregular arrangements or guide plates of irregular shape, that may be useful for treating certain pathologies, may be used without running a risk of making mistakes in the insertion of needles. Additional advantages and features of the invention will become apparent from the detail description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figures 1A and 1B show views of conventional passive brachytherapy grid or template devices currently used for guiding the insertion of needles into body tissue.
Figures 1C and 1C show conventional equipment on which template devices are usually mounted.

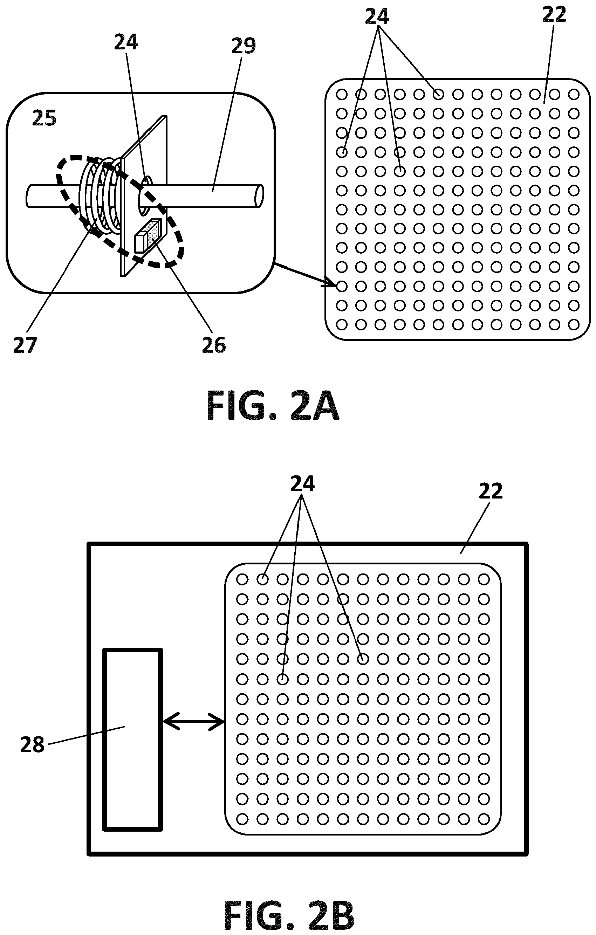

Figure 2A shows a representation of a guide plate for a system in accordance with an embodiment of the present invention. On the right, the guide plate is schematized. On the left, a detail of one of the apertures in the guide plate is shown, including a detector for detecting the presence of a needle and for determining the penetration depth of the needle into the body. Figure 2B schematically shows a guide plate for a system in accordance with an embodiment of the present invention, including control electronics for interrogating the status of each aperture.

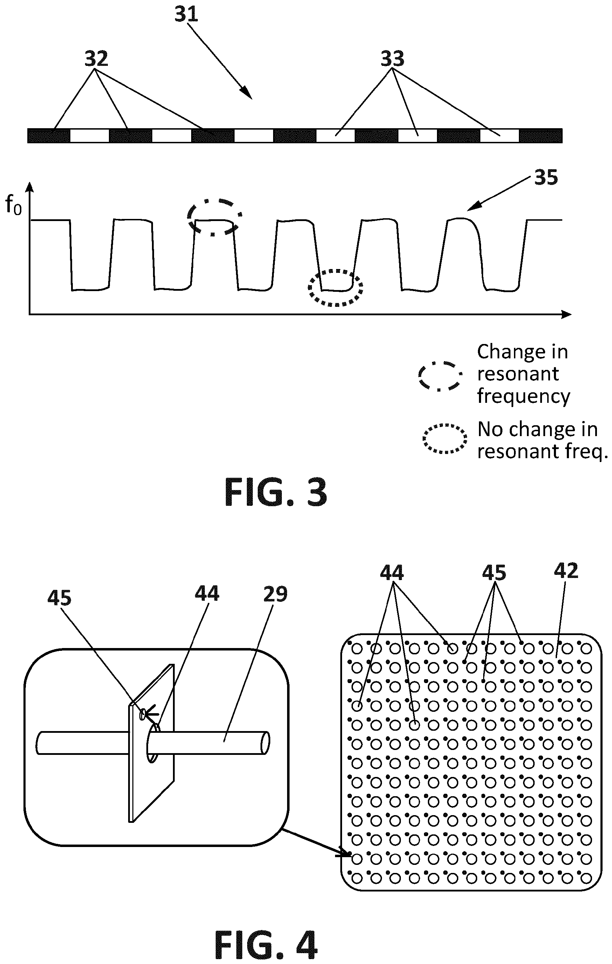

Figure 3 shows a schematic representation of a probe for a system in accordance with an embodiment of the present invention, for determining the depth at which a needle has been inserted into a body tissue through an aperture of a guide plate according to the invention. The figure also shows (bottom) a chart showing the resonant frequency of the detector associated to the aperture into which the needle is inserted, measured as the probe is inserted into the needle.
Figure 4 shows a representation of a guide plate for a system in accordance with an embodiment of the present invention. On the right, the guide plate including a plurality of light indicators is schematized. On the left, a detail of one of the apertures in the guide plate is shown, including a light indicator for indicating the aperture into which a needle should be inserted.

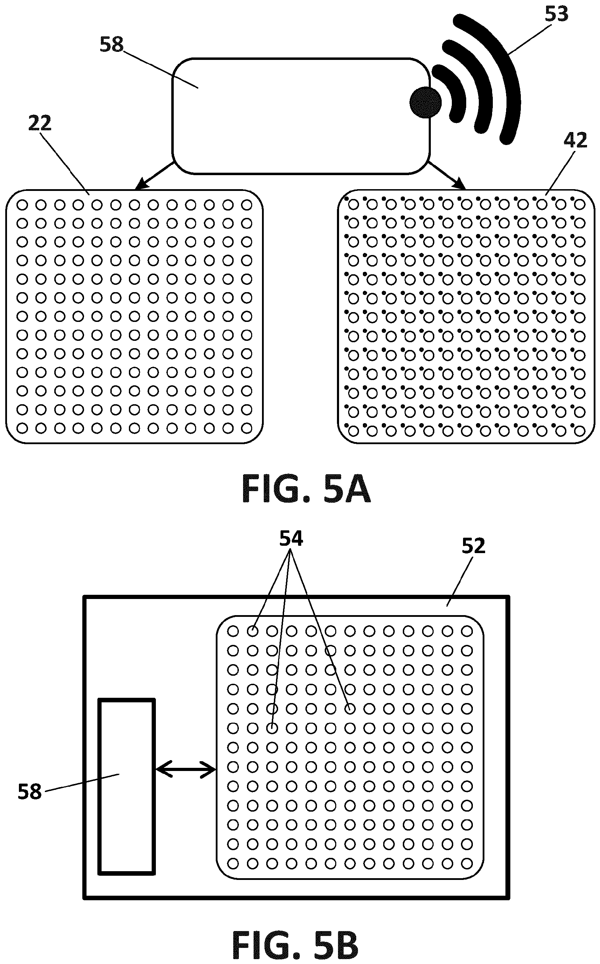

Figure 5A illustrates the detection block, illumination block and control block of a guide plate which can be used in the system according to an embodiment of the present invention. Figure 5B schematically shows a guide plate which can be used in the system in accordance with an embodiment of the present invention, including a detection block, an illumination block and control electronics for managing the detection block and the illumination block.

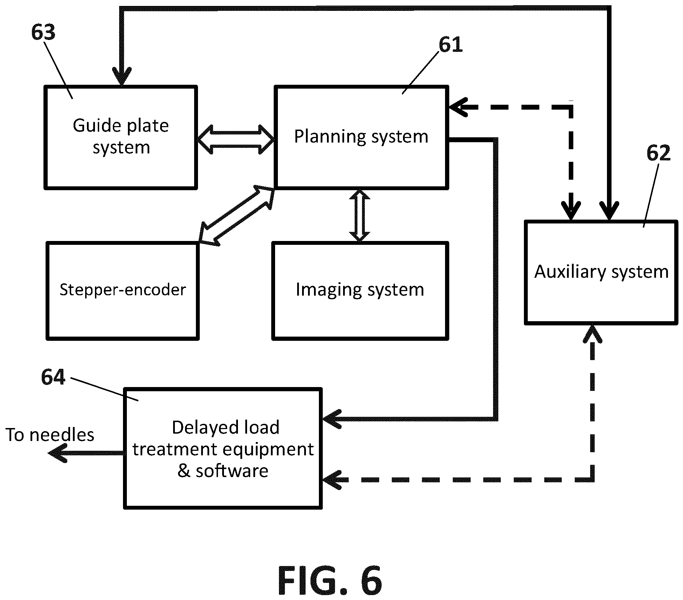

Figure 6 schematically shows a block diagram representing the equipment involved in the planning and execution of a brachytherapy treatment.

### DESCRIPTION OF A WAY OF CARRYING OUT THE INVENTION

Figure 2A (right) shows a representation of a guide plate 22 for a system in accordance with an embodiment of the present invention that may be particularly suitable for facilitating the insertion of needles into body tissue with accurate control of both position and penetration depth. The guide plate 22 includes a plurality of apertures, openings or through holes 24 for receiving the needles and guiding them towards the desired location in the body. The needles to be inserted into the apertures 24 of the guide plate 22 are usually hollow. The needles may have any length. The length of the needles may vary depending on the application (i.e. pathology and treatment). The needles may also have different gauges. The apertures 24 may form an x-y array, that is to say, apertures 24 extend in rows oriented in the x direction and in columns oriented in the y direction. There may be as many rows as columns or a different amount of rows and columns. However, apertures are not limited to this x-y arrangement; on the contrary, any other arrangement may be used, for example a non-cartesian arrangement, such as a random one. Each aperture is sized to receive one needle. The guide plate may be used in combination with a conventional passive template including a frame a fixing means or may be integrally embedded in a template during its manufacturing process. In any case, the template frame and fixing means are out of the scope of the present invention. Thus, each aperture 24 corresponds to a location (in the x and y directions when the apertures form an x-y array) on a cross section of the body part disposed substantially in parallel to the guide plate 22.

For a correct therapy, needles must be inserted through some apertures 24 according to a schedule planned prior to starting the needles insertion. That is to say, depending on the patient, pathology and treatment, some apertures will be occupied by a needle and some others will not. In order to plan the therapy, a planning system (a software program or application) is usually utilized. The planning system needs the position of the needles for performing its calculations, as well as an accurate identification of all of them (depending on the therapy, there may be more than 20 needles). The planning system uses a coordinate system for the spatial identification of the needles.

For example, in the case of HDR brachytherapy, the treatment requires the positioning of several hollow needles within and/or in the periphery of a planned target volume (PTV) of the patient, following a geometric distribution that enables, by inserting a radiation source along the inner part of the hollow needles and by stopping at planned positions and at scheduled time durations, to administer the prescribed dose with a dose distribution as homogeneous and conformed to the PTV as possible. The planning of this treatment may be as follows: First, images from the patient are obtained, from which the PTV is delimited by a physician. Then, a pre-planning stage may be performed with a computer program, which provides an optimal distribution of the needles and suggests required stops of the radioactive source. This pre-planning stage is occasionally skipped depending on the doctor's expertise. Next, the needles implant is carried out (following the pattern obtained at the pre-planning stage if this has been done; the pattern indicates where in the guide plate to implant the needles and how deep; it is remarked that not all the apertures in the guide plate receive a needle). Then, images from the patient after needles insertion are obtained, because there are often small discrepancies between the theoretical target and what is actually achieved. Next, the definitive calculation of the dose is made. Occasionally recalculation of the position of some needles may also be made, in which case verification by obtaining images is done again. Finally, the treatment starts, typically by switching on an equipment for delayed dose, such as a robot. Figure 6 schematically shows the equipment typically involved in the planning and execution of a brachytherapy treatment. As can be observed, the planning system 61 centralizes and controls the execution of the treatment.

Turning back to figure 2A, in order to prevent incorrect insertion of a needle into an aperture with respect to the scheduled plan (i.e. provided by the planning system), a needle detection means, also referred to as detection block or detection layer, is implemented in the guide plate 22. The detection means includes a set of detectors. There is one detector per aperture 24 in the guide plate 22. Figure 2A (left) shows in detail a portion of the guide plate 22 of figure 2A (right). In particular, a portion of guide plate including one of the apertures 24 of the guide plate 22 and a detector 25 associated to the aperture 24 (or substantially comprised in the aperture) is shown in figure 2A (left). A portion of needle 29 has been inserted through the illustrated aperture 24. Each detector, such as the detector 25 associated to aperture 24 as shown in figure 2A (left), is implemented as a resonant circuit disposed at each aperture 24. Each detector 25 does not obstruct or prevent the passing of a needle 29 through the aperture 24. The needle 29 is made of or comprises a material that alters the resonant frequency f₀ of the resonant circuit disposed at (associated to) each aperture 24. Alternatively, an additional element or device comprising a material that alters the resonant frequency f₀ of the resonant circuit is inserted along the hollow needle, such as a dummy element, probe or guide as disclosed later with reference to figure 3. This is for example the case when the needle 29 is not made of or does not comprise such a material. For example, this is the case of a needle made of plastic. In this case, in order to detect the presence of the needle when inserted through an aperture, an additional element or device made of or comprising such a material must accompany the needle (for example by inserting that device or element along the hollow body of the needle). The material causes a variation in the resonant frequency of the resonant circuit. So, the needle and/or an additional element or device inserted along the needle, such as a dummy element, probe or guide, must be at least partially made of such a material or of a mixture including such a material.

In embodiments of the invention, the needle 29 is a conventional needle, that is to say, a needle suitable for being inserted into an animal (including human) body tissue. Its outer surface is even and smooth. No patterns or geometrical designs are made on its outer surface.

At least one of the components of each resonant circuit 25 is disposed in the proximity of the corresponding aperture 24 in the guide plate 22 such that its nominal value is altered when the needle 29 (or a needle together with an additional element inserted along the hollow needle) traverses the aperture 24. Therefore, the resonant frequency f₀ of the resonant circuit 25 is modified due to the presence of the needle 29 when the needle 29 passes through the aperture 24. Thus, the presence of the needle 29, made of or comprising a material that varies the electric and/or magnetic field or having an additional element or device inserted along the needle, such as a dummy element (i.e. dummy steel) or a probe or guide as disclosed with reference to figure 3, made of or comprising a material that varies the electric and/or magnetic field, is electronically detected by detecting the modification in the resonant frequency f₀ caused by the varied electric and/or magnetic field. A detector 25 is thus capable of detecting the presence of the needle when there is one occupying an aperture 24. In a particular embodiment, the resonant frequency f₀ of each resonant frequency is in the band of 3 MHz (Mega Hertz, 10⁶ Hertz). In embodiments of the invention, the presence of a needle is detected by detecting variations in the resonant frequency of several kilo Hertz (kHz), such as variations between 1 and 50 kHz, or variations between 1 and 30 kHz, or variations between 2 and 20 kHz, or variations between 5 and 20 kHz.

In embodiments of the invention, the needle 29 is made of or comprises a material that alters the resonant frequency f₀ of the resonant circuit, and also an additional element or device comprising such a material (for example a dummy element, probe or guide) is inserted along the needle. Thus, the effect of the material with respect to the resonant frequency f₀ of the resonant circuit is enhanced.

In the embodiment shown in figure 2A (left), the detector 25 implemented as a resonant circuit is made of a coil 27 and a capacitive element 26. In this case, the material comprised in the needle 29 or in an additional element inserted along the needle, causes a variation in the inductance of the coil 27 when that material passes through the aperture 24 and along the longitudinal axis defined by the coil 27. For example, it causes an increase in the inductance of the coil 27. In this case, the material must be a magnetic material, for example, a ferromagnetic material. Non-limiting examples of magnetic materials that may be used are ferrites, iron, steel, and mixtures thereof. The coil 27 preferably surrounds the aperture 24, while the capacity element 26 associated to that aperture preferably has a fixed value. In this particular embodiment, the coil 27 is obtained by rolling a cable of a conductive material around the aperture 24, in such a way that the inner part of the coil coincides with the aperture 24, in such a way that a needle 29 inserted into the aperture 24 acts as magnetic core of the coil, thus modifying the inductance value of the rolled cable or coil, and therefore the resonant frequency f₀ of the detector. A non-limited example of a material of which the cable may be made is copper, but any other conductive material may be used instead. So, the needle 29 made of or comprising a magnetic material and/or having an additional element or device inserted along its inner tube, comprising a magnetic material, acts/act as magnetic core, thus permitting better coupling of the magnetic field.

In embodiments in which the resonant frequency f₀ of each resonant circuit is in the band of 3 MHz, this may be implemented with a capacitive element of around 330 pF (pico Farad, 10⁻¹² Farad) and a coil of around 7.4 µH (micro Henry, 10⁻⁶ Henry).

In embodiments of the invention, there are as many resonant circuits 25 as apertures 24 in the guide plate 22. For example, each resonant circuit 25 (or detector 25) comprises a coil 27 and a capacitive element 26.

In embodiments of the invention the resonant circuit 25 of one or more apertures 24 may be implemented differently, for example by means of the combination of one or more resistors, capacitors and/or inductors, being at least one of them sensitive to the inserted material. For example, the resonant circuit may be implemented by means of an inductor of fixed nominal value and a capacitor comprising two conductive plates (made of, for example, but not limiting, copper or aluminum) spaced by a dielectric medium (made of, for example, but not limiting, air, plastic or ceramics) disposed or configured to become parallel to the needle once the needle has been inserted through the aperture. In this case, the material comprised in the needle 29 or in an additional element inserted along the needle, causes a variation in the capacitance of the capacitor when that material passes through the aperture 24 because the electric field between the two conductive plates of the capacitor has been modified. In this case, the material must be a conductive material or a dielectric material. Non-limiting examples of materials that may be used are plastic, ceramics and mixtures thereof.

Although for simplicity of design and implementation the set of detectors is made of similar resonant circuits, one skilled in the art will understand that the guide plate may alternatively include different resonant circuits, for example, the detectors associated to some apertures may be implemented by means of a coil surrounding the aperture and a fixed capacitive element, while the detectors associated to some other apertures may be implemented differently.

In a particular embodiment, at least one of the components forming the resonant circuit 25 is disposed at one side of the guide plate 22, while at least one of the components forming the resonant circuit 25 is disposed at the other side thereof. In particular, with reference to figure 2A (left), the coil 27 is disposed at one side of the guide plate 22, while the fixed capacitive element is disposed at the other side thereof. In other embodiments of the invention, however, the two or more components forming the resonant circuit may be disposed at the same side of the guide plate.

The resonant circuits of the detection means are disposed following the disposition of the array of apertures 24 of the guide plate 22. As said, there is one resonant circuit per aperture. That is to say, when the array of apertures follows an x-y arrangement, the resonant circuits also form an x-y array, similar to the array of apertures 24 in the guide plate 22, that is to say, resonant circuits extend in rows oriented in the x direction and in columns oriented in the y direction.

The guide plate 22 also includes control electronics, not shown in figure 2A, for interrogating the resonant circuits, that is to say, for checking the resonance frequency of each resonant circuit, thus checking whether there is a needle inserted into an aperture. Each resonant circuit (therefore each aperture) is individually checked, although a group of circuits (apertures) may be simultaneously interrogated. Figure 2B schematically shows a guide plate 22 including control electronics 28 for interrogating the status of each aperture 24. The resonant circuits are connected to the control electronics 28 in order for the control electronics 28 to interrogate each resonant circuit. The control electronics 28 comprises processing means, such as a processor or microprocessor, for performing the interrogation of the resonant circuits. The connection or interconnection of each resonant circuit with the control electronics 28 may be done in different ways. For example, each resonant circuit may be individually connected to the control electronics 28, in such a way that individual interrogation of each resonant circuit is performed. Or resonant circuits may be grouped in different ways, thus performing sequential or parallel interrogation of the resonant circuits.

In a particular embodiment, in which in order to simplify the routing of paths a row-column routing configuration is used to isolate the two terminals of each resonant circuit, interrogation of resonant circuits is done sequentially. This is done as follows: The row-column of each of the two terminals of the resonant circuit is isolated and connected to an integrated circuit. This integrated circuit measures the frequency at which the resonant circuit connected to the integrated circuit resonates. Once this measurement is obtained, a next circuit is interrogated until the whole matrix is interrogated.

So far, it has been explained how the set of detectors 25 permit to determine the presence or absence of a needle in each of the apertures 24 in the guide plate 22. In other words, the detectors 25 provide the position of the needles inserted into the body tissue. Additionally, each detector 25 may be used for measuring the penetration depth of the needle inserted into the aperture associated to that detector, if any. This may be done as explained next. In order to determine how deep a needle has been inserted into the body tissue, a probe 31 may be introduced along the hollow needle. Figure 3 (top) shows a representation of a probe or guide 31 for a system in accordance with an embodiment of the present invention that may be particularly suitable for determining the depth at which a needle has been inserted into a body tissue by means of a guide plate according to the invention. As the coded probe or coded guide 31 slides along the hollow needle, the probe 31 has an influence on the resonant circuit of the detector 25 associated to the aperture into which the needle has been inserted, thus modifying the resonant frequency f₀ of the corresponding detector. The probe 31 is a coded probe. In the embodiment shown in figure 3 (top), the probe 31 is coded by using different materials along its length, thus providing different properties depending on the amount of probe inserted into the needle. Assuming that the probe 31 is inserted into a needle -in turn inserted into the body tissue by means of the guide plate 22 already described-, up to the distal end of the inner part of the needle, the detected resonant frequency f₀ represents the amount of needle inserted into the body tissue. In the embodiment shown in figure 3 (top), the coding scheme is implemented by alternating portions of a first material 32 with portions of a second material 33.

In embodiments of the invention, the first material is a material that varies the electric and/or magnetic field, and therefore it is electronically detected by detecting the modification in the resonant frequency f₀ caused by the varied electric and/or magnetic field.

In embodiments of the invention in which the resonant circuit is implemented by means of a coil 27 and a capacitive element 26 (see for example figure 2A (left), the probe 31 is coded by providing different magnetic properties along its longitudinal axis. The probe 31 is coded by using different materials along its length, thus providing different magnetic properties depending on the amount of probe inserted into the needle. Assuming that the probe 31 is inserted into a needle -in turn inserted into the body tissue by means of the guide plate 22 already described-, up to the distal end of the inner part of the needle, the detected resonant frequency f₀ represents the amount of needle inserted into the body tissue. In this embodiment, the first material 32 is a ferromagnetic material and the second material 33 is a dielectric material. The first material 32 provides substantial ferromagnetic response, while the second material 33 provides substantially null ferromagnetic response. In other words, the first material 32 is capable of modifying the resonant frequency f₀ of the detector associated to the aperture into which the needle is inserted, while the second material 33 does not modify the resonant frequency f₀ of that detector. Non-limiting examples of ferromagnetic material used for implementing the first material 32 are: ferrite, iron, surgical steel (biocompatible steel) and mixtures thereof. Non-limiting examples of non-ferromagnetic material 33 used for implementing the second material are: plastic, ceramic, some metals, such as aluminum or copper, and combinations thereof.

Figure 3 (bottom) shows a chart showing the resonant frequency f₀ of the detector associated to the aperture of the guide plate into which a needle is inserted, measured as the probe 31 is inserted into the needle (that is to say, the resonant frequency is measured at the detector as the probe is sliding along the hollow tube of the needle). When a portion of material 32 providing ferromagnetic response passes through or along the detector, a change in the resonant frequency of the detector is recorded, while when a portion of material 33 not providing ferromagnetic response passes through or along the detector (see for example the detector 25 in figure 2A (left)), no change in the resonant frequency of the detector is recorded with respect to the resonant frequency already modified by the needle (assuming the needle is made of or comprising a ferromagnetic material). It is remarked that the resonant frequency of the detector has already been slightly modified by inserting the needle into the aperture if the needle is made of or comprises a ferromagnetic material, or by inserting an additional element (i.e. dummy element) having ferromagnetic material along the needle, or by both. Thus, by counting the amount of transitions and applying a conversion into length units (for example, millimeters or micrometers (microns)), the amount of length of the needle that has been inserted into the body tissue is then calculated.

In embodiments of the invention, the probe or guide 31 has outer surface substantially even and smooth. For example, the probe or guide 31 may have the shape of a cylinder, either hollow or solid. The cylinder may be made of different materials as already stated.

In a particular embodiment, the probe 31 is made of equidistant sections or portions of ferromagnetic and dielectric materials 32, 33, as shown in figure 3 (top). In a particular embodiment, each portion has a length varying between 0,2 and 20 mm, preferably between 0,5 and 10 mm, more preferably between 1 and 5 mm. Because the portions of different materials have the same length and are alternating (ferromagnetic, dielectric, ferromagnetic, dielectric...), the introduction of the probe 31 into the needle and its sliding along the needle creates a periodic pulse train 35, as shown in figure 3 (bottom). By multiplying the number of pulses by the length of each section or portion of the probe 31, the amount of length of probe 31 that has crossed the detector 25 can be calculated.

For example, for a needle, made of steel, inserted in position (x=5, y=C), its measured resonance frequency is f_{1_5C} = 3.212 MHz, while the frequency of the next position (without needle) is f_{0_5D}= 3.206 MHz. In a next step, when a coded probe 31 made of alternate portions of ferrite powder and plastic is slipped through the needle up to its end, the resonance frequency varies from f_{1_5C} = 3.212 MHz to f_{2_5C} = 3.219 MHz when a portion of probe having a material providing ferromagnetic response (ferrite powder) passes along the longitudinal axis of the coil, and the resonance frequency becomes again f_{1_5C} = 3.212 MHz when a portion of probe having a material providing no ferromagnetic response (plastic) passes along the longitudinal axis of the coil. By counting the amount of transitions from f_{1_5C} to f_{2_5C}, quantification of the penetration depth can be assessed. The probe 31 is only inserted into the needle for assessing its depth. The probe 31 can then be taken out of the hollow needle. So, by analyzing the sequence of changes in the resonant frequency obtained by sliding the probe 31, the amount of probe 31 inserted into the needle is obtained. Thus, the distance between the distal end of the needle and the detector is obtained.

With respect to the detection of the presence of a needle, if for example a dummy steel is inserted through the needle already inserted in position (x=5, y=C), its resonance frequency becomes f'_{1_5C}= 3.221 MHz, making the detection process easier (more accurate) because 3.221 MHz differs from 3.206 MHz in 15 KHz, while 3.212 MHz differs from 3.206 MHz only in 6 KHz.

The coded probe or guide 31 may be inserted into the needle prior to the insertion of the needle through an aperture, in which case both the needle and the coded probe are simultaneously inserted through the aperture. The variation in the resonant frequency may be correctly tracked. However, in preferred embodiments, the needle is inserted through the aperture first (and poked into the tissue) and then the coded probe is inserted along the hollow needle and up to the end of the needle, which has already been fixed to the tissue. The estimation of the depth of the needle is thus correctly calculated. This procedure prevents undesired (and undetectable) movement of the probe within the needle while the needle is being poked into the tissue, for example. This procedure provides the additional advantage of double checking the variations in the resonant frequency when the coded probe is removed from the needle, because substantially the same sequence of resonant frequencies will be tracked, thus checking the penetration depth and improving the precision of the estimation.

Next, another embodiment of the system of the invention is disclosed, with reference to figure 4, in which the guide plate permits to assist the person inserting the needles with a visual guidance at the insertion area in order to still increase accurate control of needle position and prevention of human mistakes. The guide plate providing this visual guidance may be embodied in combination with the embodiment described with reference to figures 2A, 2B and 3 or with independence from that embodiment. So, the features described in relation to former figures 2A, 2B and 3 may be comprised in embodiments having the additional features described in relation to figure 4.

Figure 4 (right) shows a representation of a guide plate 42 for a system in accordance with an embodiment of the present invention that may be particularly suitable for facilitating the insertion of needles into body tissue with increased control of position and prevention of human mistakes. All the features of the guide plate 22 disclosed regarding figures 2A-2B may apply as well to the guide plate shown in figure 4.

As already disclosed, for a correct therapy, needles must be inserted through some apertures 24 according to a schedule planned prior to starting the therapy and therefore prior to the needles insertion. Even though the position of inserted needles is detected by means of the detectors already disclosed, and therefore the presence of a wrong needle is detected and/or the absence of a required needle is detected, it is desirable that the person inserting the needles does not make a mistake when inserting the needles, in order not to hurt or disturb the patient or not to delay the therapy. In order to prevent incorrect insertion of a needle into an aperture due to incorrect identification on the actual plate or template of the target aperture as indicated in the planned schedule, an illumination means (also referred to as illumination block or illumination layer) is implemented in the guide plate 42. The illumination means provides the person inserting the needles into the guide plate 42 with real time information. The illumination means shows the aperture(s) in the guide plate 42 through which a needle must be inserted. The illumination means provides a set of light indicators 45. Each light indicator 45 is configured to provide a light signal visible by a person standing in the proximity of the guide plate 42. The light signal emitted by each light indicator 45 may be visible for a person situated with respect to the plate 42 at a distance equal to or less than 10 meters. There is one light indicator 45 per aperture 24 in the guide plate 42. All the apertures have an associated light indicator. That is to say, when the array of apertures 24 in the guide plate 42 forms an x-y arrangement, the light indicators 45 form an x-y array as well, that is to say, light indicators 45 extend in rows oriented in the x direction and in columns oriented in the y direction.

Figure 4 (left) shows in detail a portion of the guide plate 42 of figure 4 (right). In particular, a portion of guide plate including one of the apertures 44 of the guide plate 42 is shown in figure 4 (left). A portion of needle 29 has been inserted through the illustrated aperture 44. Each light indicator 45, such as the light indicator 45 associated to aperture 44 as shown in figure 4 (left), may be implemented as a light pilot next to each aperture 44 or within the aperture, provided that the emitted light is perceptible from said distance at which a person inserting needles is standing. In a particular embodiment, the light indicators 45 are a plurality of light emitting diodes (LEDs), each LED being disposed next to a corresponding aperture 44. At least one LED is associated to one aperture. All the apertures have at least one LED associated thereto. In another embodiment, the light indicators may be implemented as a plurality of transparent tubes, each of them being disposed within the corresponding aperture. In this case, for example a LED may be attached to one side of each transparent tube, allowing the light to propagate towards both edges.

In a particular embodiment, the illumination means is implemented on a layer made of printed circuit board (PCB) disposed in front of the main body forming the guide plate 42 and attached or embedded thereto. In embodiments in which a guide plate comprises both detectors 25 for determining the presence and penetration depth of needles and light indicators 45 for guiding the person inserting the needles, detectors 25 and light indicators 45 may be disposed on a same layer of PCB or on different layers thereof.

In an example of use of the light indicators 45, a light indicator associated to an aperture through which a needle must be inserted according to a planned schedule emits a light signal prior to inserting the needle. This is controlled, for example, by management equipment which is out of the scope of the present invention. The light signal may be, for example, a permanent green light. Once the needle is correctly inserted in the corresponding aperture (that is to say, the needle has been inserted into the aperture indicated in the schedule), the light signal emitted by the light indicator is modified, for example by emitting a different intensity, or a different color, such as yellow, or a blinking light instead of a permanent one, in order to indicate the user that the needle has been correctly inserted, so that he/she may go on with other needles. If, on the contrary, the needle is not correctly inserted in the corresponding aperture, the light signal emitted by the light indicator is modified, for example by emitting a different color, such as red, and/or increasing intensity and/or blinking. So, the light indicators 45 serve the purpose of indicating the apertures in the guide plate through which needles must be inserted and providing the user with feedback on the correct or erroneous insertion of needles. So, the process of needle insertion is speeded up while its correctness is optimized.

The guide plate 42 also includes control electronics, not shown in figure 4, for managing the switching on and off of the light indicators. The control electronics for the light indicators may be similar to the control electronics for interrogating the status of each aperture, shown in figure 2B. In embodiments of the invention, the control electronics comprises processing means, such as a processor or a microprocessor. In embodiments of the invention in which the guide plate comprises both detectors 25 for determining the presence and penetration depth of needles and light indicators 45 for guiding the person inserting the needles, the same control electronics may manage or control both the detectors and the light indicators. This is illustrated in figure 5A, wherein control electronics 58 is implemented as processing means and controls the detectors (in guide plate 22) and the light indicators (in guide plate 42). The illustrated guide plates 22, 42 may be implemented in different layers (for example of PCB) or in a same layer (for example a PCB). Non-limiting examples of processing means are a microprocessor, a microcontroller, a digital signal processor or any other equipment with capacity of processing.

It may be generally referred to as a processor. Figure 5B schematically shows a guide plate 52 including control electronics 58 for interrogating the detectors and light indicators of each aperture 54.

Next, a method for inserting needles into a body, for example in relation to a medical treatment, such as a radiation therapy, for example brachytherapy, is explained in relation to the already disclosed figures. The method does not fall within the scope of the invention. First, a needle is inserted along one of the plurality of apertures 24 of a guide plate. Associated with each aperture, for example, in the proximity of each aperture, there is a detector. The detector comprises a resonant circuit for detecting the presence of the needle once the needle has been inserted into the aperture to which the detector is associated. Upon interrogation triggered and control by control electronics, the detector detects the resonant frequency produced when the needle is inserted into the aperture. So far, the presence of a needle in an aperture has been detected. Such detection is performed every time a needle is inserted into an aperture. In order to enhance the accuracy of the detection, and depending on the material of the needle and on the type of resonant circuit implemented in the detectors, an additional element (such as a dummy elongated element, probe or guide) may be inserted along the hollow tube of the needle. After a needle has been inserted and therefore its presence has been detected, a probe 31 is inserted within the needle and slid along the inner hollow body of the needle, up to the distal end of the needle. The probe is used for measuring, together with the detector associated to the aperture into which the needle has been inserted, how deep the needle has been inserted into the body tissue. While the probe is inserted, changes in the resonant frequency produced in the detector by the probe are tracked. In order to provoke those changes in the resonant frequency of the detector, the probe 31 has along its longitudinal axis a plurality of portions of a first material 32 and a plurality of portions of a second material 33. The portions of the first material 32 alternate with portions of the second material. The first material 32 is capable of modifying the resonant frequency of the detector associated to the aperture into which the needle is inserted, while the second material 33 does not modify the resonant frequency of the detector. So, by analyzing the sequence of changes recorded in the resonant frequency as the probe moves forward, the penetration depth of the needle is determined. If the alternate portions of first material 32 and second material 33 are of equal length, the total penetration of needle into the body tissue can be easily calculated.

The method also comprises a stage for indicating a user, prior the insertion of a needle, in which aperture said needle should be inserted and, after insertion of a needle, whether the needle has been correctly inserted according to a schedule previously planned. In other words, immediate, visual verification of the correctness of the needle insertion can be made. This is done by means of a light indicator associated to each aperture in the guide plate. The light indicator is for example emitting a light signal indicative that a needle must be inserted into the aperture associated to the light indicator. And when the needle is inserted, the light signal is modified in order for the user to check whether the insertion is correct or not. The light indicators are for example LEDs.

The method is managed by control electronics in charge of interrogating the detectors, either individually or in groups (simultaneously), and of controlling the light indicators.

Apart from controlling or managing the needle detection and penetration across the resonant circuits in the detection block or detection layer 22 and the lighting of light indicators in the illumination block or illumination layer 42, the control electronics 28, 58 also provides interface means or connection means with any other equipment involved in the therapy (a non-limiting example of possible connections with other equipment is schematically shown in figure 6). This interface means may be wired or wireless and is schematized in figure 5 with reference sign 53. So, control electronics 58 (control block of the guide plate) comprises processing means implementing algorithms for detection, illumination, communication and management with other equipment involved in the therapy. Control electronics 58 includes at least one computing means configured to perform the control of the different layers or blocks with independence from the technology used to implement these blocks. For example, parameters like the resonant frequency of each resonant circuit and detected variations in the resonant frequency due to the presence of needle or probe are transparent for the user. It also manages the light indicators. Control electronics 58 thus provide the user with an indication of which needles have been inserted and how deep they have been inserted within the body tissue, and whether the needles have been correctly inserted (that is to say, whether they have been inserted in the right aperture). In sum, it provides the user with instant information of the detection and penetration depth of needles and current illumination associated to needles to be inserted and to correct/incorrect insertion of needles already inserted. In a particular embodiment, each block (for example detection block 22 and illumination block 42) communicates with other blocks by means of a digital bus and the control electronics 58 implements communication interfaces with other equipment different from the guide plate. The communication interfaces are preferably wireless links.

So, the guide plate 22, 42 performs the following functionalities by means of the control electronics: Management of operation mode of the guide plate, such as individual or simultaneous detection of needles and measurement of their penetration depth and how information is feedback to other processes or equipment. Management of query policies in the resonant circuits (such as individual queries, sequential queries, adaptive queries, etc.). Management of light indicators (for example indicating the status in which the guide plate is operating and/or the detected errors in the location of needles). Possible status may be: a LED is illuminating an aperture waiting for the insertion of a needle; or the position of a needle has been correctly detected; or an aperture is waiting for the insertion of a probe in order to assess the penetration depth of a needle. Management and implementation of different communication protocols used to provide the data from the guide plate to other equipment (for example to a computer for visualization of control or to a dosimetry planning tool).

In embodiments of the invention, the disclosed system (referred to as 63 in figure 6) is connected to an auxiliary system 62 that improves its interface. The auxiliary system 62 comprises a processor, such as a computer, running a specific software that communicates with the disclosed system 63 and exhibits at least the capabilities of: selective illumination of LEDs to guide the medical staff during the needles' insertion, according to the programmed dosimetry plan; recording the real position in which the needle has been detected; recording the penetration depth of each detected needle; communicating the actual needles' positions and penetration depths to the dosimetry software (referred to as 64 in figure 6) for a possible adjustment of the dosimetry plan.

In this text, the term "comprises" and its derivations (such as "comprising", etc.) should not be understood in an excluding sense, that is, these terms should not be interpreted as excluding the possibility that what is described and defined may include further elements, steps, etc. In this text, the terms "device" and "node" have been interchangeably used; further, the terms "multiplicity" and "plurality" have been interchangeably used. In embodiments of the invention, the auxiliary system 62 may be part of the planning system 61, the planning system 61 providing the corresponding interface with the guide plate system 63.

The invention is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

## Claims

1. A system for inserting needles into a body, comprising:
a guide plate (22, 42, 52) comprising a plurality of apertures (24), each aperture being configured to receive a needle to be inserted into a body, wherein at least some apertures of said plurality of apertures (24) have a respective detector (25) associated thereto, each detector (25) comprising a resonant circuit for detecting the presence of a needle inserted into a respective aperture (24), said resonant circuit being configured to modify its resonant frequency when the needle is inserted into the aperture;
the system being **characterized in that** it further comprises:
a probe (31) for measuring, together with the detector (25) comprised in an aperture, the penetration depth of a needle inserted into said aperture (24), each detector (25) being configured to track changes in its resonant frequency produced when said probe (31) is inserted along the needle in turn inserted into the aperture (24); and
control electronics (28) for interrogating each detector (25) and for determining the presence or absence of a needle in a corresponding aperture (24) and for determining the penetration depth of a detected needle from said tracked changes in the resonant frequency of a corresponding detector.

2. The system of claim 1, wherein said probe (31) comprises along its longitudinal axis a plurality of portions of a first material (32) and a plurality of portions of a second material (33), wherein portions of said first material (32) alternate with portions of said second material (33), said first material (32) being capable of modifying the resonant frequency of the detector associated to the aperture into which the needle is inserted, said second material (33) not modifying the resonant frequency of said detector, the penetration depth of the needle being determined by counting the number of times the resonant frequency of said detector is modified as the probe (31) slides along the needle until the probe (31) reaches the distal end of the needle.

3. The system of claim 2, wherein said needle to be received by an aperture (24) comprises said first material (32).

4. The system of claim 2, wherein said needle to be received by an aperture (24) is configured to receive an additional element or device comprising said first material (32)

5. The system of any of claims 2-4, wherein said probe (31) comprises alternate portions of said first material (32) and said second material (33), said portions being of equal length.

6. The system of any preceding claim, wherein each resonant circuit comprises a coil (27) and a capacitive element (26), wherein the nominal value of either said coil (27) or said capacitive element (26) is altered when a needle traverses said aperture (24).

7. The system of claim 6, wherein the nominal value of said coil (27) is altered when a needle traverses said aperture (24), the nominal value of said capacitive element (26) remaining fixed.

8. The system of claims 2 and 7, wherein said first material (32) is a magnetic material and said second material (33) is a material with no magnetic response.

9. The system of any preceding claim, wherein said guide plate (22, 42, 52) further comprises a light indicator (45) associated to each aperture in the guide plate (22, 42, 52), each light indicator (45) being configured to indicate a user of the system, prior the insertion of a needle, in which aperture said needle should be inserted and/or, after insertion of a needle, whether the needle has been correctly inserted according to a schedule previously planned, and/or configured to emit a light signal prior to inserting a needle and a modified light signal once a needle has been inserted, said modified light signal being indicative of the correctness or incorrectness location of the inserted needle.

10. The system of claim 9, wherein said light indicator (45) associated to each aperture in the guide plate (22, 42, 52) is at least one light emitting diode (LED).

11. The system of any preceding claim, wherein said guide plate (22, 42, 52) further comprises interface means for connection with other equipment.

12. The system of any preceding claim, wherein said guide plate (22, 42, 52) is comprised in a brachytherapy template device.

## Patentansprüche

1. System zum Einführen von Nadeln in einen Körper, umfassend:
eine Führplatte (22, 42, 52),umfassend eine Vielzahl von Öffnungen (24), wobei jede Öffnung ausgestaltet ist, eine in einen Körper einzuführende Nadel zu aufzunehmen,
wobei zumindest einige Öffnungen der Vielzahl von Öffnungen (24) einen jeweiligen assoziierten Detektor (25) aufweisen, wobei jeder Detektor (25) einen Schwingkreis zum Detektieren des Vorliegens einer in eine jeweilige Öffnung (24) eingeführte Nadel umfasst, und der Schwingkreis ausgestaltet ist, seine Resonanzfrequenz zu modifizieren,
wenn die Nadel in die Öffnung eingeführt ist;
wobei das System **dadurch gekennzeichnet ist, dass** es weiter umfasst:
eine Sonde (31) zum Messen, gemeinsam mit dem in einer Öffnung umfassten Detektor (25), der Eindringtiefe einer in die Öffnung (24) eingeführten Nadel, wobei jeder Detektor (25) ausgestaltet ist, Änderungen in seiner Resonanzfrequenz nachzuverfolgen, welche erzeugt werden, wenn die Sonde (31) entlang der Nadel eingeführt ist, welche wiederum in der Öffnung (24) eingeführt ist; und
Steuerelektronik (28) zum Befragen eines jeden Detektors (25) und zum Bestimmen des Vorliegens oder Nicht-Vorliegens einer Nadel in einer entsprechenden Öffnung (24) sowie zum Bestimmen der Eindringtiefe einer detektierten Nadel auf Basis der nachverfolgten Änderungen in der Resonanzfrequenz eines entsprechenden Detektors.

2. System nach Anspruch 1, wobei die Sonde (31) entlang ihrer Längsachse eine Vielzahl von Abschnitten eines ersten Materials (32) und einer Vielzahl von Abschnitten eines zweiten Materials (33) umfasst, wobei Abschnitte des ersten Materials (32) mit Abschnitten des zweiten Materials (33) alternieren, wobei das erste Material (32) fähig ist eines Modifizierens der Resonanzfrequenz des mit der Öffnung assoziierten Detektors, in welche die Nadel eingeführt ist, das zweite Material (33) die Resonanzfrequenz des Detektors nicht modifiziert, die Eindringtiefe der Nadel bestimmt wird durch Zählen der Anzahl der Male, bei welchen die Resonanzfrequenz des Detektors modifiziert wird, während die Sonde (31) entlang der Nadel gleitet, bis die Sonde (31) das distale Ende der Nadel erreicht.

3. System nach Anspruch 2, wobei die durch eine Öffnung (24) aufzunehmende Nadel das erste Material (32) umfasst.

4. System nach Anspruch 2, wobei die durch eine Öffnung (24) aufzunehmende Nadel ausgestaltet ist, ein(e) zusätzliche(s), das erste Material (32) umfassende(s) Element oder Vorrichtung aufzunehmen.

5. System nach Anspruch 2-4, wobei die Sonde (31) alternierende Abschnitte des ersten Materials (32) und des zweiten Materials (33) umfasst, wobei die Abschnitte gleicher Länge sind.

6. System nach einem beliebigen vorhergehenden Anspruch, wobei jeder Resonanzkreis eine Spule (27) und ein kapazitives Element (26) umfasst, wobei der nominelle Wert entweder der Spule (27) oder des kapazitiven Elements (26) geändert wird, wenn eine Nadel die Öffnung (24) durchtritt.

7. System nach Anspruch 6, wobei der nominelle Wert der Spule (27) geändert wird, wenn die Nadel die Öffnung (24) durchtritt, wobei der nominelle Wert des kapazitiven Elements (26) fixiert ist.

8. System nach Ansprüchen 2 und 7, wobei das erste Material (32) ein magnetisches Material ist, und das zweite Material (33) ein Material ohne magnetische Wirkung ist.

9. System nach einem beliebigen vorhergehenden Anspruch, wobei die Führplatte (22, 42, 52) weiter einen mit jeder Öffnung in der Führplatte (22, 42, 52) assoziierten Lichtanzeiger (45) umfasst, wobei jeder Lichtanzeiger (45) ausgestaltet ist, einem Verwender des Systems anzuzeigen vor Einführen einer Nadel, in welche Öffnung die Nadel eingeführt werden soll, und/oder nach Einführen einer Nadel, ob die Nadel korrekt eingeführt worden ist gemäß einem im Voraus geplanten Ablauf, und/oder ausgestaltet ist zum Emittieren eines Lichtsignals vor einem Einführen einer Nadel sowie eines modifizierten Lichtsignals, sobald eine Nadel eingeführt worden ist, wobei das modifizierte Lichtsignal anzeigt, ob der Ort der eingeführten Nadel korrekt oder inkorrekt ist.

10. System nach Anspruch 9, wobei der mit jeder Öffnung in der Führplatte (22, 42, 52) assoziierte Lichtanzeiger (45) zumindest eine lichtemittierende Diode (LED) ist.

11. System nach einem beliebigen vorhergehenden Anspruch, wobei die Führplatte (22, 42, 52) weiter Schnittstellenmittel zum Verbinden mit weiterer Ausrüstung umfasst.

12. System nach einem beliebigen vorhergehenden Anspruch, wobei die Führplatte (22, 42, 52) in einer Brachytherapieschabloneneinrichtung umfasst ist.

## Revendications

1. Système destiné à insérer des aiguilles dans un corps, qui comprend :
une plaque de guidage (22, 42, 52) qui comprend une pluralité d'ouvertures (24), chaque ouverture étant configurée pour recevoir une aiguille à insérer dans un corps, dans lequel au moins certaines ouvertures de ladite pluralité d'ouvertures (24) possèdent un détecteur respectif (25) associée à celles-ci, chaque détecteur (25) comprenant un circuit résonant pour détecter la présence d'une aiguille insérée dans une ouverture respective (24), ledit circuit résonant étant configuré pour modifier sa fréquence de résonance lorsque l'aiguille est insérée dans l'ouverture ;
le système étant **caractérisé en ce qu'**il comprend en outre :
une sonde (31) pour mesurer, avec le détecteur (25) compris dans une ouverture, la profondeur de pénétration d'une aiguille insérée dans ladite ouverture (24), chaque détecteur (25) étant configuré pour suivre les changements de sa fréquence de résonance produits lorsque ladite sonde (31) est insérée le long de l'aiguille à son tour insérée dans l'ouverture (24) ; et
une électronique de commande (28) destinée à interroger chaque détecteur (25) et à déterminer la présence ou l'absence d'une aiguille dans une ouverture correspondante (24) et à déterminer la profondeur de pénétration d'une aiguille détectée à partir desdits changements suivis de fréquence de résonance d'un détecteur correspondant.

2. Système selon la revendication 1, dans lequel ladite sonde (31) comprend, le long de son axe longitudinal, une pluralité de parties d'un premier matériau (32) et une pluralité de parties d'un second matériau (33), dans lequel les parties dudit premier matériau (32) alternent avec les parties dudit second matériau (33), ledit premier matériau (32) étant capable de modifier la fréquence de résonance du détecteur associé à l'ouverture dans laquelle l'aiguille est insérée, ledit second matériau (33) ne modifiant pas la fréquence de résonance dudit détecteur, la profondeur de pénétration de l'aiguille étant déterminée en comptant le nombre de fois où la fréquence de résonance dudit détecteur est modifiée lorsque la sonde (31) coulisse le long de l'aiguille jusqu'à ce que la sonde (31) atteigne l'extrémité distale de l'aiguille.

3. Système selon la revendication 2, dans lequel ladite aiguille à recevoir par une ouverture (24) comprend ledit premier matériau (32).

4. Système selon la revendication 2, dans lequel ladite aiguille à recevoir par une ouverture (24) est configurée pour recevoir un élément ou un dispositif supplémentaire comprenant ledit premier matériau (32).

5. Système selon l'une quelconque des revendications 2 à 4, dans lequel ladite sonde (31) comprend des parties alternées dudit premier matériau (32) et dudit second matériau (33), lesdites parties étant de longueur identique.

6. Système selon l'une quelconque des revendications précédentes, dans lequel chaque circuit résonant comprend une bobine (27) et un élément capacitif (26), dans lequel la valeur nominale de ladite bobine (27) ou dudit élément capacitif (26) est modifiée lorsqu'une aiguille traverse ladite ouverture (24).

7. Système selon la revendication 6, dans lequel la valeur nominale de ladite bobine (27) est modifiée lorsqu'une aiguille traverse ladite ouverture (24), la valeur nominale dudit élément capacitif (26) restant fixe.

8. Système selon les revendications 2 et 7, dans lequel ledit premier matériau (32) est un matériau magnétique et ledit second matériau (33) est un matériau sans réponse magnétique.

9. Système selon l'une quelconque des revendications précédentes, dans lequel ladite plaque de guidage (22, 42, 52) comprend en outre un témoin lumineux (45) associé à chaque ouverture dans la plaque de guidage (22, 42, 52), chaque témoin lumineux (45) étant configuré pour indiquer à un utilisateur du système, avant l'insertion d'une aiguille, l'ouverture dans laquelle ladite aiguille doit être insérée et/ou, après l'insertion d'une aiguille, si l'aiguille a été correctement insérée selon un scénario préalablement planifié, et/ou configuré pour émettre un signal lumineux avant d'insérer une aiguille et un signal lumineux modifié une fois qu'une aiguille a été insérée, ledit signal lumineux modifié indiquant l'exactitude ou l'inexactitude de l'emplacement de l'aiguille insérée.

10. Système selon la revendication 9, dans lequel ledit témoin lumineux (45) associé à chaque ouverture dans la plaque de guidage (22, 42, 52) est au moins une diode électroluminescente (LED).

11. Système selon l'une quelconque des revendications précédentes, dans lequel ladite plaque de guidage (22, 42, 52) comprend en outre un moyen d'interface destiné à la liaison avec un autre équipement.

12. Système selon l'une quelconque des revendications précédentes, dans lequel ladite plaque de guidage (22, 42, 52) est comprise dans un dispositif de gabarit de curiethérapie.
